# EUROPÄISCHE PATENTSCHRIFT

(11) **EP 0 557 738 B1**
(45) Veröffentlichungstag und Bekanntmachung des Hinweises auf die Patenterteilung: **07.07.1999**
(21) Anmeldenummer: 93101459.1
(22) Anmeldetag: 30.01.1993
(51) Int. Cl.: B07C 5/342, B07C 5/34, G01N 33/44

(54) **Verfahren und Vorrichtung zur Unterscheidung von Kunststoffteilen sowie Verwendung des Verfahrens zur Aussonderung wiederverwertbarer Kunststoffteile aus Industrie- und/oder Hausmüll**
Method and apparatus for separation of plastic components and use of the method for extraction or recyclable plastic components from industrial and/or domestic waste
Procédé et dispositif pour distinguer des pièces en matière plastique et l'usage du procédé pour séparer des pièces recyclables en matière plastique de déchets industriels et/ou ménagers

(30) Priorität: 25.02.1992 DE 4205630
(43) Veröffentlichungstag der Anmeldung: 01.09.1993
(73) Patentinhaber: TZN Forschungs- und Entwicklungszentrum Unterlüss GmbH, D-29345 Unterlüss (DE)
(72) Erfinder: Neff, Helmut, Dr. rer. nat., W-3102 Hermannsburg (DE); Laukemper, Jürgen, Dr. rer. nat., W-3100 Celle (DE); Giet, Johannes, Dr., W-3104 Unterlüss (DE); Meyer, Walter, W-3104 Unterlüss (DE); Steffen, Peter, Dr. rer. nat., W-3100 Celle (DE)
(74) Vertreter: Behrend, Rainer

(56) Entgegenhaltungen:
- AT-B- 384 563
- GB-A- 2 229 809
- US-A- 4 644 163

## Beschreibung

Die Erfindung betrifft ein Verfahren zur Unterscheidung von Kunststoffteilen aus unterschiedlichem Material. Die Erfindung bezieht sich ferner auf eine Vorrichtung zur Durchführung des Verfahrens und eine Verwendung des Verfahrens zur Aussonderung wiederverwertbarer Kunststoffteile aus Industrie- und/oder Hausmüll.

Der Wiederverwertung, insbesondere des Plastikanteils von Industrie- und Hausmüll, kommt bereits derzeit eine große Bedeutung zu, die in Zukunft noch wachsen wird. Um zu einem wirtschaftlich nutzbaren Anteil zu gelangen, muß der Plastikanteil aus dem Müll ausgesondert und die einzelnen Kunststoffsorten stoffspezifisch sortiert werden. Soweit der Anmelderin bekannt, wird die Aussonderung derzeit in der Regel manuell vorgenommen, wobei die stoffspezifische Trennung durch visuelle Unterscheidung von stoff- bzw. herstellerspezifischen Formen erfolgt (z.B. sind Joghurtbecher aus Polyethylen und Haarwaschmittelbehälter aus PVC etc. hergestellt).

Ein derartiges Verfahren weist aber eine Reihe von Nachteilen auf. Zum einen ist es langfristig gesehen unwirtschaftlich und weist einen vergleichsweise geringen Wirkungsgrad durch hohe Belastung des Personals in gesundheitsgefährdender Umgebung auf. Zum anderen gewährleistet die manuelle Sortierung nicht in allen Fällen eine stoffspezifische Trennung der Kunststoffteile, die für die Herstellung eines reinen, hochwertigen Granulates erforderlich ist.

In der GB-A-2229809 werden mögliche Verfahren zur Aussonderung von wiederverwertbarem Müll beschrieben. So wird in dieser Druckschrift beispielsweise vorgeschlagen, die zu sortierenden Teile mit einer Strahlung geeigneter, auch unterschiedlicher Wellenlänge zu bestrahlen. Die von den Teilen dann hindurchgelassene oder reflektierte Strahlung wird dazu verwendet, die Teile mittels Spektrophotometer zu identifizieren.

Ferner wird in der GB-A-2229809 bereits offenbart, daß zur Klassifizierung wiederverwertbarer Teile diese mittels einer Kamera auch auf ihre Form und Farbe hin untersucht werden können.

Aus der AT-A-384563 ist eine Vorrichtung zum Sortieren von kleinen Gegenständen (z.B. Diamanten) bekannt, bei der die zu sortierenden Teile ebenfalls mit einer Strahlung geeigneter Wellenlänge bestrahlt werden und anschließend die von den Teilen erzeugte Fluoreszenzstrahlung mittels eines Detektors mit nachgeschaltetem Bildverstärker analysiert wird, wobei ein Vergleich mit gespeicherten Daten erfolgt.

Der vorliegenden Erfindung liegt die Aufgabe zugrunde, ein Verfahren der eingangs erwähnten Art derart weiterzuentwickeln, daß eine automatische Sortierung mit stoffspezifischer Trennung unterschiedlicher Kunststoffe im Haus- oder Industriemüll möglich ist. Ferner soll eine Vorrichtung zur Durchführung des Verfahrens offenbart werden.

Die vorstehende Aufgabe wird erfindungsgemäß durch die Merkmale der Ansprüche 1 und 4 gelöst. Besonders vorteilhafte Ausgestaltungen der Erfindung ergeben sich aus den Unteransprüchen.

Vorrichtungen zur Durchführung des erfindungsgemäßen Verfahrens sind in den Ansprüchen 4 und 5 offenbart.

Anspruch 6 bezieht sich auf eine Verwendung des Verfahrens zur Aussonderung wiederverwertbarer Kunststoffteile aus Industrie- und/oder Hausmüll.

Der Erfindung liegt also im wesentlichen der Gedanke zugrunde, die unterschiedlichen spektralen Absorptions- bzw. Emissionseigenschaften der Kunststoffe auszunutzen. Bei Beleuchtung mit Licht führt dieses, bei Benutzung eines bildgebenden Systems (Kamera), zu stoffspezifischen Kontrastunterschieden. Anhand gesetzter Parameter und geometrischer Merkmale können somit Aussagen über das jeweilige Material gewonnen werden.

Weitere Einzelheiten und Vorteile der Erfindung werden im folgenden anhand von zwei Ausführungsbeispielen und mit Hilfe von Figuren näher erläutert. Es zeigen:
- Fig.1: eine Emissionsmeßvorrichtung; und
- Fig.2: eine Transmissionsmeßvorrichtung zur Durchführung des erfindungsgemäßen Verfahrens.

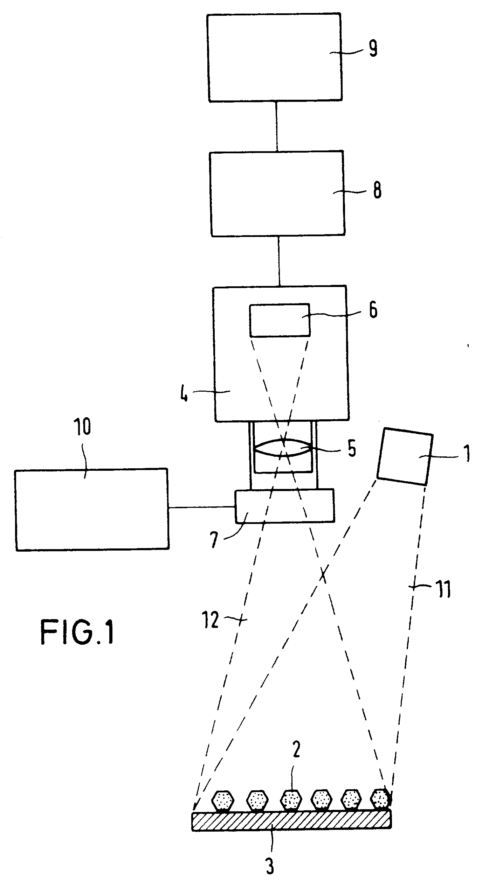

In Fig.1 ist mit 1 eine Strahlungsquelle bezeichnet, die die zu selektierenden Kunststoffteile 2 auf einem Förderband 3 beleuchtet. Das entsprechende Licht ist mit dem Bezugszeichen 11 versehen. Ist gewährleistet, daß die Kunststoffteile auf eine Temperatur aufgeheizt sind, die deutlich oberhalb der Raumtemperatur liegt, so kann auf den Einsatz der Strahlungsquelle auch verzichtet werden. Aufgrund ihrer unterschiedlichen chemischen Zusammensetzung emittieren die Plastikteile 2 Licht bei verschiedenen Wellenlängen mit unterschiedlicher Intensität.

Als Empfangsvorrichtung wird ein bildgebendes System (Kamera) 4 verwendet. Die Kamera 4 nimmt das emittierte Licht 12 auf und bildet es mit Hilfe einer geeigneten Optik 5 auf einem Detektor 6 ab. Durch die Bewegung des Förderbandes ist ein 1-dimensionaler Zeilendetektor als Sensor geeignet. Es kann jedoch auch ein 2-dimensionaler Matrixdetektor oder ein Einelementdetektor mit einer Scaneinrichtung eingesetzt werden.

Da die Intensität des von den Plastikteilen 2 emittierten Lichtes 12 von der chemischen Zusammensetzung des Kunststoffes und von der Wellenlänge abhängt, ist es notwendig, daß die Kamera 4 das Licht 12 wellenlängenselektiv empfängt, um aus den Intensitätsunterschieden auf das Kunststoffmaterial zu schließen.

Zur Wellenlängenselektion werden z.B. Interferenzfilter 7 eingesetzt. Die Filter 7 können entweder vor der Strahlungsquelle 1 montiert sein, so daß die Beleuchtung bereits schmalbandig erfolgt, oder im Bereich der Kamera 4, z.B. vor der Optik 5 oder vor dem Detektor 6.

Aus den Intensitätsunterschieden (Kontrast) zwischen den bei mindestens zwei verschiedenen Wellenlängen λ1 und λ2 gemessenen emittierten Licht des zu selektierenden Kunststoffes wird dann auf das Material der jeweiligen Kunststoffteile 2 geschlossen. Dabei werden die Wellenlängen λ1 und λ2 vorzugsweise derart gewählt, daß sich ein maximaler Kontrast ergibt. Dieses bedeutet, daß die Wellenlänge λ1 derart gewählt wird, daß sich bei vorgegebenem Beobachtungswinkel eine maximale Intensität des emittierten Lichtes ergibt (sog. Emissionsmaximum). Demgegenüber wird die Wellenlänge λ2 derart gewählt, daß sich ein Emissionsminimum ergibt, die Emissionsintensität also einen Minimum erreicht.

Die Umschaltung der Wellenlängen kann z.B. dadurch erfolgen, daß die Filter 7 auf einer rotierenden Scheibe angeordnet sind. Die Umlauffrequenz muß mit der Bildaufnahmefrequenz des Detektors 6 synchronisiert sein, so daß während einer Bildaufnahme sich genau ein Interferenzfilter 7 im Strahlengang befindet. Eine andere Möglichkeit der Wellenlängenselektion besteht in der Verwendung eines durchstimmbaren Filters. In diesem Fall wird die Änderung der Wellenlänge durch eine elektrische Ansteuerung 10 des Filters 7 hervorgerufen.

Die vom Detektor 6 erzeugten Signale werden in einer Signalverarbeitungselektronik 8 verarbeitet, digitalisiert und anschließend über eine Bildverarbeitungs-Software ausgewertet.

Die aufgenommenen Bildsequenzen zeigen, je nach der über das aktive Filter 7 eingestellten Wellenlänge, materialspezifische Kontrastunterschiede der Kunststoffteile 2, aus denen die Materialart bestimmt werden kann. Das Ergebnis wird über eine geeignete Schnittstelle 9 ausgegeben.

Da Kontrastunterschiede auch aufgrund von Temperaturunterschieden der Kunststoffteile 2 hervorgerufen werden, ist sicherzustellen, daß die Kunststoffteile zum Zeitpunkt der Bildaufnahme sich auf annähernd der gleichen Temperatur befinden. Ebenso sollte das Förderband aus einem Material bestehen, das einen gleichbleibenden Kontrast in den einzelnen Wellenlängenbereichen gewährleistet.

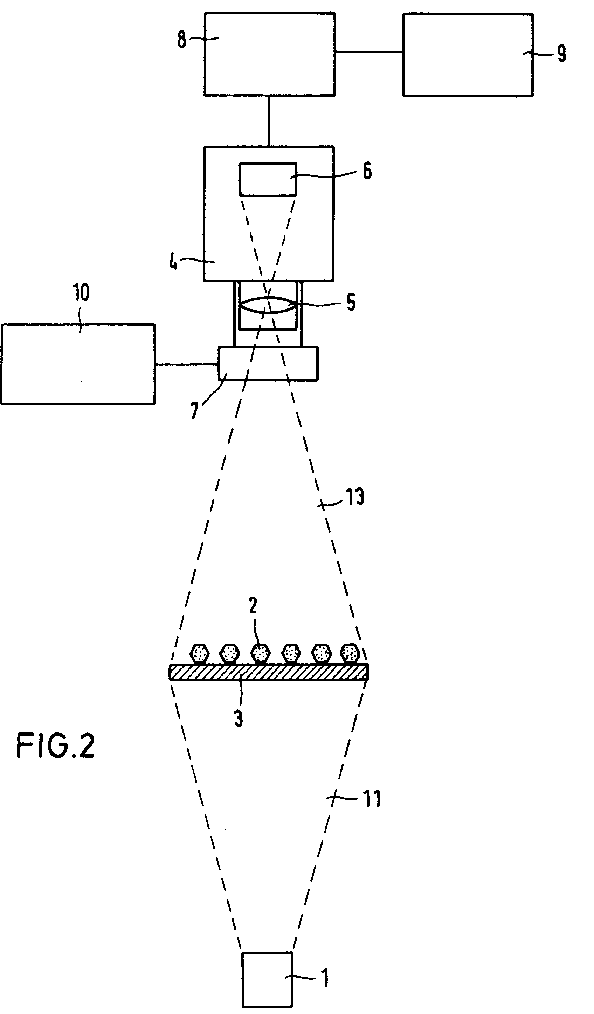

In Fig. 2 ist ein weiteres Ausführungsbeispiel der Erfindung dargestellt. Dabei sind die gleichen Komponenten wie in Fig.1 mit den gleichen Bezugszeichen versehen. Die Beleuchtung mit Licht 11 erfolgt hier derart, daß die Strahlungsquelle 1 der Kamera 4 gegenüberliegt und die Kunststoffteile 2 und das Förderband 3 vom Licht durchstrahlt werden (Transmissionsmessung). Der wesentliche Unterschied zu der in Fig.1 dargestellten Meßanordnung besteht also darin, daß anstelle der Emissionsunterschiede bei verschiedenen Wellenlängen die material- und wellenlängenselektive Transmission der Kunststoffteile 2 ausgenutzt wird, die ebenfalls zu einem Kontrastunterschied in den Bildern, aufgenommen bei unterschiedlichen Wellenlängen, führt, woraus die Kunststoffart ermittelt werden kann. Auch in diesem Fall werden vorteilhafterweise die Wellenlängen λ1 und λ2 derart gewählt, daß sich ein maximaler Kontrast ergibt.

Bei diesem Verfahren muß das Förderband 3 aus einem Material bestehen, das bei den verwendeten Wellenlängen eine gute Lichttransmission zeigt.

### Bezugszeichenliste

- 1: Strahlungsquelle
- 2: Kunststoffteile
- 3: Förderband
- 4: Empfangsvorrichtung, bildgebendes System, Kamera
- 5: Optik
- 6: Detektor
- 7: optisches Filter
- 8: Signalverarbeitungs- und Bildverarbeitungsvorrichtung
- 9: Ein-/Ausgabe Schnittstelle
- 10: Filteransteuerung
- 11: Licht
- 12: emittiertes Licht
- 13: durch die Kunststoffteile hindurchgelassenes Licht

## Patentansprüche

1. Verfahren zur Unterscheidung von Kunststoffteilen (2) aus unterschiedlichem Material mit folgenden Merkmalen:
- es wird Licht (11) auf die zu untersuchenden Kunststoffteile (2) gestrahlt oder die Kunststoffteile (2) werden auf eine Temperatur aufgeheizt, die oberhalb der Raumtemperatur liegt;
- es wird das von den Kunststoffteilen (2) emittierte und/oder bei einer Transmissionsmessung hindurchgelassene Licht (12;13) bei mindestens zwei verschiedenen Wellenlängen (λ1, λ2) mittels mindestens eines bildgebenden Systems empfangen;
- aus den Intensitätsunterschieden zwischen den bei verschiedenen Wellenlängen aufgenommenen Bildern wird auf das Material der jeweiligen Kunstoffteile (2) geschlossen.

2. Verfahren nach Anspruch 1, **dadurch gekennzeichnet,** daß die Wellenlängen (λ1, λ2) derart gewählt werden, daß sich ein maximaler Kontrast ergibt.

3. Verfahren nach Anspruch 1 oder 2, **dadurch gekennzeichnet,** daß zur Emissions- und/oder Transmissionsmessung Licht des Infrarotbereiches des elektromagnetischen Spektrums verwendet wird.

4. Vorrichtung zur Unterscheidung von Kunststoffteilen (2) aus unterschiedlichem Material mit
a) einer Strahlungsquelle (1) zur Bestrahlung der Kunststoffteile (2) mit Licht bzw. einer Einrichtung zur Aufheizung der Kunststoffteile (2) auf eine Temperatur oberhalb der Raumtemperatur;
b) mindestens einem bildgebenden System (4) zum wellenlängenselektiven Empfang des von den Kunststoffteilen emittierten und/oder hindurchgelassenen Lichtes bei mindestens zwei verschiedenen Wellenlängen (λ1, λ2);
c) einer Bildverarbeitungsvorrichtung (8) zur Ermittlung der Intensitätsunterschiede der bei verschiedenen Wellenlängen aufgenommenen Bilder und der sich aus diesen Werten ergebenden Kunststoffsorte.

5. Vorrichtung nach Anspruch 4, dadurch gekennzeichnet, daß entweder vor dem bildgebenden System (4) oder hinter der Strahlungsquelle (1) ein durchstimmbares optisches Filter (7) angeordnet ist.

6. Verwendung des Verfahrens nach Anspruch 1 zur Aussonderung wiederverwertbarer Kunststoffteile (2) aus Industrie- und/oder Hausmüll.

## Claims

1. Method for distinguishing plastic components (2) of different materials, with the following characteristics:
- light (11) is directed onto the plastic components (2) to be examined, or the plastic components (2) are heated to a temperature which is above the room temperature;
- the light (12;13) emitted by the plastic components (2) and/or transmitted in a transmission measuring operation is received at least at two different wave lengths (λ1, λ2) by means of at least one imaging system;
- from the intensity differences between the images received at the different wave lengths the material of the plastic components (2) under examination is determined.

2. Method in accordance with Claim 1, characterised by the fact that the wave lengths (λ1, λ2) are selected to ensure a maximum contrast.

3. Method in accordance with Claim 1 or 2, characterised by the fact that light within the infrared range of the electromagnetic spectrum is used for the emission and/or transmission measuring operation.

4. Apparatus for distinguishing plastic components (2) of different materials, with
(a) a radiation source (1) for directing light onto the plastic components and with a device for heating the plastic components (2) to a temperature above the room temperature;
(b) at least one imaging system (4) for wave length selective reception at least at two different wave lengths (λ1, λ2), of the light emitted and/or transmitted by the plastic components;
(c) an image processing device (8) for determining the intensity differences of the images received at different wave lengths and for assessing the type of plastic indicated by these values.

5. Apparatus in accordance with Claim 4, characterised by the fact that a variable-frequency optical filter (7) is provided in front of the imaging system (4) or behind the radiation source (1).

6. Use of the method according to Claim 1 for separating recyclable plastic components (2) from industrial and/or domestic waste.

## Revendications

1. Procédé destiné à distinguer des éléments en matière plastique (2) en matériaux différents présentant les caractéristiques suivantes:
- de la lumière (11) est rayonnée sur les éléments en matière plastique (2) à analyser ou les éléments en matière plastique (2) sont chauffés à une température qui est supérieure à la température ambiante;
- la lumière (12; 13) émise par les éléments en matière plastique (2) et/ou qui est passée lors d'une mesure de transmission est reçue pour au moins deux longueurs d'ondes différentes (λ1, λ2), au moyen d'au moins un système de production d'images;
- à partir des différences d'intensité entre les images prises à des longueurs d'ondes différentes, on conclut quant au matériau des éléments en matière plastique (2) concernés.

2. Procédé selon la revendication 1, caractérisé en ce que les longueurs d'ondes (λ1, λ2) sont choisies de manière à donner un contraste maximal.

3. Procédé selon la revendication 1 ou 2, caractérisé en ce que pour la mesure d'émission et/ou de transmission on utilise la lumière du domaine infrarouge du spectre électromagnétique.

4. Dispositif destiné à distinguer des éléments en matière plastique en matériaux différents comportant :
a) une source de rayonnement (1) destinée à irradier les éléments en matière plastique (2) de lumière ou un dispositif de chauffage des éléments en matière plastique à une température supérieure à la température ambiante;
b) au moins un système (4) de production d'images pour la réception avec sélection des longueurs d'ondes de lumière émise par les éléments en matière plastique et/ou traversée pour deux longueurs d'ondes différentes (λ1, λ2);
c) un dispositif de traitement d'images (8) destiné à déterminer les différentes intensités des images prises pour différentes longueurs d'ondes et du type de la matière plastique résultant de ces valeurs.

5. Dispositif selon la revendication 4, caractérisé en ce qu'un filtre optique (7) variable est disposé soit devant le système (4) de production d'images, soit derrière la source de rayonnement (1).

6. Mise en oeuvre du procédé selon la revendication 1, pour trier des éléments en matière plastique (2) récupérables provenant de déchets industriels et/ou d'ordures ménagères.
